# EUROPEAN PATENT APPLICATION

(11) **EP 2 578 558 A1**
(43) Date of publication of application: **10.04.2013**
(21) Application number: 12186633.9
(22) Date of filing: 28.09.2012
(51) Int. Cl.: C05F 17/00, B01D 53/58, C05C 3/00

(54) **Process for the recycling of the nutrient elements used by crops**

(30) Priority: 06.10.2011 IT MI20111822; 11.04.2012 IT MI20120587
(71) Applicant: Agroittica Acqua e Sole S.p.A., 20124 Milano (IT)
(72) Inventor: Natta, Giuseppe, 27010 Giussago (PV) (IT); Donati, Gianni, 27010 Giussago (PV) (IT)
(74) Representative: Minoja, Fabrizio

(57) **Abstract**

Process for the recycling of nutrient elements and for the preparation of fertilisers from organic waste coming from the cycle of production and consumption of foods by subjecting the wastes to an anaerobic fermentation in order to obtain a digestate and biogas; extracting ammonia from the digestate with a gas; absorbing the ammonia with a fluid containing sulphuric or phosphoric acid for the production of ammonium sulphate / phosphate. The remaining digestate can also be used as a fertiliser.

## Description

The object of the present invention is a process for the recycling of nutrient elements and for the preparation of fertilisers from organic substrates from waste coming from the cycle of production and consumption of foods. More particularly the invention relates to a new method of extraction of the ammonia with the production of several types of fertilisers suitable for the different phases of cultivation.

### State of the art

The problem of the future non-availability of nutrient elements, in particular of the main components of fertilisers (nitrogen, phosphorus or potassium compounds), derives from the fact that every harvest removes from the soil a quantity of nutrient elements proportional to own weight. The waste deriving from the use of the products of the harvest containing the nutrient elements in general does not return to the soil, wherefrom it has been taken, unlike what occurs in the natural cycle and in the agricultural productions of the past, where the harvest was mainly used at the land where it was produced. This consolidated practice was interrupted due to the urbanisation and industrialisation of the activities downstream of agricultural cultivations, so that the waste of the food cycle is produced and disposed of in territories in general far from those wherein the crop was produced.

It is therefore necessary to reconstitute, after every harvest, the reserve of nutrient elements removed by means of the distribution in the soil of chemical fertilisers, which use scarce non-renewable resources, such as the deposits of potassium and phosphorous compounds and energy reserves for the production of nitrogen compounds.

Environmental problems are added to this problem which are particularly felt for the nitrogen compounds and for the phosphorous ones. In fact, since the quantities removed from the cultivated soils are replaced by nutrient elements coming from other regions, the waste which derives from the cycle of production and consumption of the foods, from the harvest and from human nutrition, ends up in the environment, causing the known problems of pollution.

Recently, however, the possibility of concentrating the waste, coming from the food cycle in the form of purification biological sludge and the separation of the food refuse contained in urban waste has allowed the provision for recovery of nutrient elements in a more concentrated form and to transport them, after possible treatment, onto soils which can be cultivated also far from the place where they are generated. In order to reduce the environmental impact caused by the odours emitted by the rottable waste, aerobic stabilisation processes are used, as in the production of compost from the fraction of the urban waste constituted by food refuse and in the stabilisation of the urban purification sludge and of the sewage of animal breeding farms. This stabilisation is also performed by means of systems of anaerobic digestion, by means of which the rottable organic compounds, the source of unpleasant odours, are transformed into biogas. These processes produce materials which, if they have suitable features, often regulated by law, can be intended for disposal on agricultural land. This disposal is not configured as an alternative system to the use of chemical fertilisers, since it is difficult to integrate it with the time demands of periodical agricultural activities. It is not moreover possible to ensure health safety, both due to the presence of possible pathogens coming from waste of faecal origin and due to the possibility of being contaminated during the operations of storage and distribution.

Many processes for obtaining biogas from the digestible components of said waste have been described, but the digestion is carried out in diluted conditions and it is often followed by the separation of solid materials which can be composted aerobically and of an aqueous waste to be disposed of, never in any case for the purpose of obtaining concentrated liquid fertilisers, free from pathogens and which can be stored in closed tanks to avoid the possible contamination thereof, without the production of other waste.

Moreover the presence of ammonia in the digester prevents the completion of the digestion, slowing down the process of anaerobic fermentation and reducing the yields.

Numerous patents relate to the anaerobic digestion of similar substrates, intended only for the production of biogas, and not for the recovery of the nutrient elements by means of the production of fertilisers.

The methods described to date therefore do not solve the following problems:
- the difficulty of mixing of fluids concentrated to the limit of pumpability in order to have a distribution of nutrients in the mass;
- the simultaneous need to eliminate the ammonia which, in concentrated substrate conditions, increases with the continuation of digestion until it prevents anaerobic fermentation;
- the need to supply heat in an economical manner in order to maintain large volumes at high and homogeneous temperatures, above 55°C, in order to guarantee, during the thermophilic fermentation, also the pasteurisation against pathogens;
- the need to obtain as product of the digestion exclusively two fluid materials which can be used as fertilisers, with high concentration of dry substance and which can be stored in closed tanks to avoid possible contamination.

CA 1102019 discloses a combined system of mixing and heating which uses a vertical conduit with lining placed at the centre of the digester provided with means for the pumping of the suspension from below upwards.

DE 10354063 claims the digestion preferably in batches at high temperatures (70-85°C) and in a vacuum (10-80 kpa) for the stripping of the ammonia and the absorption of the ammonia contained in the biogas exiting the digester at a lower temperature (40°C) in a suspension of calcium sulphate possibly containing sulphuric acid.

EP 0563434 proposes the mixing of the sludge in fermentation with a system of pipes positioned on the base of the digester in the zones wherein the sludge itself and the biogas are pumped in sequence.

GB 2457681 proposes as system of mixing a mobile arm rotating on the base of the digester actuated by a flow of gas.

US 4824571 divides the base of the digester into zones provided with systems of distribution of the gas to be activated in sequence.

US 2007102352 performs the stripping of the ammonia from the product of the digester with hot air heated for example by burning biogas and treating the effluent gas with a biofilter.

In a similar manner WO 2010098343 heats the liquid coming from the fermenter to strip the ammonia and WO 2011018269 uses hot exhausted gases in a column where it neutralises the ammonia extracted with sulphuric acid.

As can be observed, all these methods offer solutions intended for the production of biogas but not for the production of fluid fertilisers having a high content of dry substance in order to be able to be stored and distributed economically.

Other known processes, in addition to those described above, after the production of biogas, separate a solid phase, which can be used as fertiliser, from a diluted liquid, which can optionally be disposed of on the soil, but incompatible with a replacement use of the fertilisation, this use requiring stocks of excessive dimensions and fertilisers to be distributed in a restricted time.

It also has to be considered that the organic substrates are in general of faecal origin, possibly concentrated in the form of sludge of biological purification plants and therefore contain, in addition to the nutrient elements (N, P, K, etc.), rottable organic substances which generate unpleasant odours, possible pathogens, which determine health problems, and have a variable physical state (solid, sludgy or liquid). This, together with the health and environmental problems, makes the production of homogeneous and odourless fertilisers not contaminated by pathogens, their storage and their distribution, difficult.

Examples of organic substrates comprise the sewage of livestock farms, the organic waste of the agricultural and food industry, the biological sludge of urban purifiers, the clean wet fraction of urban waste.

In this scenario the manufacture of a system which allows recycling in the soils from which the crop has been taken, of the waste of the cycle of production and consumption of foods, would solve both the problem of the availability of these resources and the environmental one connected to the dispersion in the environment of excess nutrient elements.

### Description of the invention

A process has now been found which allows the limitations and the disadvantages of the known processes to be overcome.

The process which is the object of the invention allows the production of two fertilisers, a mixed organic fertiliser, to be used before sowing, and a nitrogen fertiliser, for example ammonia phosphate or sulphate, which can also be used with cultivations underway, which uses the waste coming from the cycle of the production and consumption of foods (organic substrates) and allows on a regional basis the recycling on agricultural land of the nutrient elements removed by the harvest, solving the health, environmental and logistical problems in a sustainable and economical manner, it being possible to use for the energy requirement of the process exclusively the energy which can be obtained by means of the digestion of said organic substrates in conditions of temperature suitable for obtaining the pasteurisation of the fertilisers obtained.

The process which is the object of the present invention allows a regional system to be set up which is able to re-establish a sustainable agricultural activity, allowing the recycling of the nutrient elements, which traditional agriculture performed on a local scale, also on a regional scale.

The process which is the object of the present invention operates as a continuous process and solves the main problems of the mixing of the digester on high volumes of the digester itself and in the presence of viscous and concentrated fluids. It also solves the problem of the stripping of ammonia in the presence of vapour-liquid equilibria unfavourable due to the presence of liquid phase reactions, of the carbon dioxide and of the slowness of the phenomena of transport of material and of heat linked to the viscosity of the fluids.

The mixing of the solids is performed by means of high recycle of the digested product on the digester and placing in close contact said recycle with the feed in a special apparatus which we shall call mixer.

The stripping of the ammonia is performed by means of high recycle of the biogas produced in the digester or of an inert gas which is placed in contact with the recycle of the digested product in a special apparatus which we shall call evaporator.

The ammonia is extracted from said recycle of gas by means of scrubbing with a stream containing phosphoric or sulphuric acid and transformed into ammonia phosphate or sulphate in an absorber.

The digester is insulated and maintained at a temperature of around 55°C while the evaporator operates at temperatures slightly higher than 55°C or according to a preferred embodiment up to temperatures of around 80°C.

The biogas produced is used in order to produce electrical energy in internal combustion engines and the heat necessary for the process is supplied using the thermal energy released by said motors with the hot combustion gases and with the cooling water.

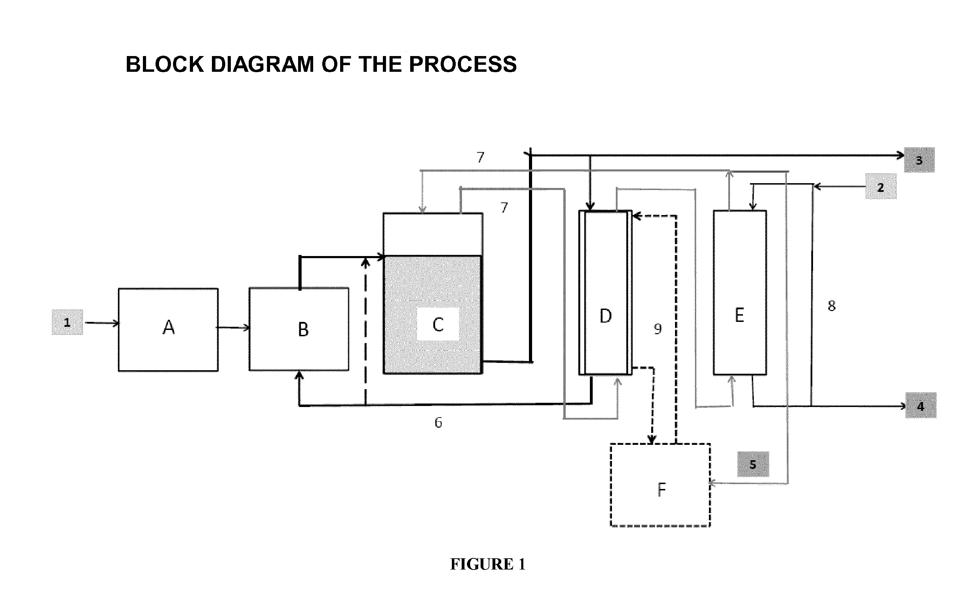

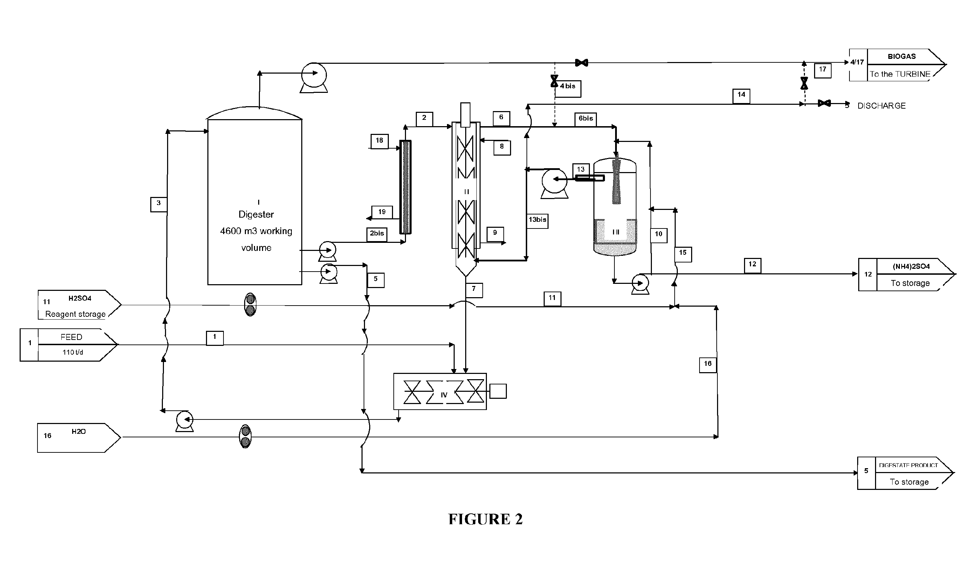

Figs. 1 and 2 describe two possible embodiments of the process according to non-limiting example diagrams and which are differentiated one from the other mainly by different systems of recycling and use of the gas of stripping of the ammonia and by different operating conditions of the evaporator and of the absorber of the ammonia.

In these drawings the reference numeral 1 denotes the waste from the production and consumption of foods (organic substrates with possible water). The reference numeral 11 denotes the line of feeding of sulphuric/phosphoric acid and possible water for the production of the fertiliser 2. Reference numerals 5 and 12 denote respectively the fertilisers 1 and 2 at storage. The biogas is denoted by reference numeral 4. The numeral 8 denotes the vapour or hot fluid from cogeneration plant.

A denotes the section of reception of the organic substrates.

I denotes the digester - insulated tank for ensuring a retaining time typically of 20 days.

II denotes an evaporator with heat exchange for the stripping of the ammonia and for the heating of the circulating fluid.

III denotes the apparatus of scrubbing with sulphuric or phosphoric acid of the ammonia, while the cogeneration plant is denoted by the letter F.

IV represents a mixer/homogeniser of the substrates fed with the hot concentrate of the evaporator II.

According to a first embodiment, the process of the invention comprises:
a) collection of said organic substrates in the solid, liquid or sludgy state in one or more vessels in a closed environment in a vacuum connected to a system of deodorisation of the air and their movement with gantry cranes (A);
b) fine dispersion in the mixer (IV) of the organic substrates fed in a concentrated fluid circulating on the digester and which constitutes the Fertiliser 1 produced in the digester;
c) anaerobic digestion as a continuous process in a digester (I) of the organic substrates dispersed at temperatures higher than that of pasteurisation, in general higher than 55°C, for a sufficient time for the thermophilic digestion of the same organic substrates with production of biogas;
d) extraction as a continuous process at temperatures slightly higher than 55°C and preferably between 55 and 60°C in the evaporator (II) during anaerobic digestion, from said circulating Fertiliser 1, of the ammonia, using as extraction fluid a recirculation of the biogas produced in the digester and preferably supplying the heat necessary for the process using the residual heat coming from internal combustion engines;
e) continuous extraction in the scrubbing apparatus (III) from the biogas exiting from the evaporator of the ammonia, transferred to the biogas from the concentrated fluid, by a second fluid consisting of a concentrated solution of an ammonium salt, which constitutes the Fertiliser 2, whereto acid of adequate concentration is added in a continuous process, sufficient for neutralising the ammonia extracted, the temperature of this fluid being close to that of the evaporator and such as to avoid or control the transfer of water from the first to the second circulating fluid and vice versa;
f) use of part of the biogas exiting from the scrubbing, free from ammonia and recirculated to the digester, as fuel in internal combustion engines in order to produce electrical energy and to supply all the energy required by the process;
g) storage of the Fertiliser 1 in a tank of sufficient capacity for supplying the Fertiliser 1 in the period of fertilisation before sowing, and storage of the Fertiliser 2 in a tank of sufficient capacity for supplying the Fertiliser 2 in the period of fertilisation also subsequent to sowing.

It is pointed out that in this embodiment the biogas exiting from the digester and recycled to the same digester passes through the evaporator and the scrubbing of the gas.

Operating the evaporator at temperatures higher than 60°C would entail an excessive evaporation of the water with consequent concentration of the solids and excessive consumptions of heat in the evaporator moreover not available from internal combustion engines.

It has however been found that operating in the evaporator at temperatures higher than 55°-60°C offers appreciable advantages due to the fact that the equilibria of ammonia are more favourable for the purpose of the stripping all due to the almost complete elimination from the liquid phase to the gas phase of the carbon dioxide.

Therefore according to an alternative embodiment described in the diagram of Fig. 2, the removal of the ammonia from the digester is performed by evaporating partially the digested product extracted from the digester in an evaporator operating at temperatures higher than 55°C of the digester and preferably from 65 to 95°C, without requiring the high energy consumptions linked to the evaporation of high quantities of water.

In order to maintain the evaporation of the ammonia high without evaporating the water, the process is performed by means of an evaporator and of an apparatus for the scrubbing with sulphuric or phosphoric acid wherein the gas exiting from the evaporator after scrubbing is recycled to the evaporator itself.

Said gas can be steam, biogas saturated with water or also an inert gas in such a way as to avoid a successive water evaporation required by the vapour-liquid equilibria and therefore high energy consumptions for evaporating ammonia.

The recycling of the biogas between digester and evaporator plus acid scrubbing is in this way eliminated and replaced with an internal recycling between evaporator and acid scrubbing.

The plant can preferably operate at atmospheric pressure or at a slight excess pressure.

According to the alternative embodiment and with reference to Fig. 2, the process of the invention comprises:
a) collection of said organic substrates in the solid, liquid or sludgy state in one or more vessels in a closed environment in a vacuum connected to a system of deodorisation of the air and their movement with gantry cranes (A);
b) fine dispersion in the mixer (IV) of the organic substrates in a hot concentrated fluid exiting from the evaporator and in the possible hot waters recovered from the plant to be fed to the digester c), compensating in this way the heat dispersions of the digester itself;
c) anaerobic digestion as a continuous process in a digester (I) of the organic substrates dispersed in b) at temperatures higher than that of pasteurisation, in general around 55°C, for a sufficient time for the thermophilic digestion of the same organic substrates with production of biogas;
d) extraction as a continuous process, during the anaerobic digestion c), of the digested product and of the biogas produced by the digester c);
e) feeding of part of the digested product to an evaporator (II) wherein the same digested product is heated to temperatures preferably comprised between 65 and 80°C, in which only a part of the water is evaporated and the carbon dioxide and most of the ammonia are released in vapour phase in the steam or saturated vapours without ammonia coming from the acid scrubbing of the evaporated stream f);
f) continuous extraction in the scrubbing apparatus (III) from the vapour or from the steam saturated gases coming from the evaporator d) of the ammonia, in a fluid circulating on the acid scrubbing consisting of a concentrated solution of an ammonium salt, whereto acid of adequate concentration is added in a continuous process, sufficient for neutralising the ammonia extracted in order to produce ammonia phosphate or sulphate in solution, the temperature of this fluid being close to that of the evaporator d);
g) the biogas extracted from the digester and still containing ammonia can be used directly as fuel in internal combustion engines in order to supply all the energy required by the process or fed to the acid scrubbing where it is purified of the ammonia and placed in cycle between evaporator and acid scrubbing before being purged from the cycle, optionally condensed for the vapour drawn and sent to the engines;
h) storage of the digested product, defined as Fertiliser 1, in a tank of sufficient capacity for supplying the Fertiliser 1 preferably in the period of fertilisation before sowing, and storage of the ammonia phosphate or sulphate, defined as Fertiliser 2 in a tank of sufficient capacity for supplying the Fertiliser 2 in the period of fertilisation also subsequent to sowing.

### Detailed description of the invention

### First embodiment

The organic substrates 1, whatever their physical state (solid, sludgy or liquid), are collected in one or more ponds in a closed environment under moderate vacuum A connected with a system of deodorisation of the air, preferably a biofilter (not shown in the drawing). They are moved, preferably by means of robotised gantry cranes, and roughly mixed one with the other.

Said substrates, treated in this way, are fed into a mixer IV which allows their micronisation and mixing with a part of the Fertiliser 1 produced in the digester I which, as is known, is more fluid and pumpable, having transformed a part of the solids into biogas, obtaining in this way more favourable effects: the possibility of feeding also solid substrates and therefore of operating concentrated, a close mixing and dispersion of the micro-organisms in the mass fed and the possible elimination of lumps and solid bodies. For this operation it is possible to use pulpers or ball mills, the latter being indicated for substrates containing solids to be ground such as for example the stones in fowl droppings.

The Fertiliser 1 is treated in the digester I constituted by a tank, suitable for guaranteeing the necessary retaining time, typically 20 days, for the thermophilic digestion of the organic substrates dispersed at a temperature higher than that required by pasteurisation, in general 55°C, and for the production of biogas. At said digester I arrive the flow of substrates exiting from the phase of dispersion IV and the recirculation of the biogas purified of the ammonia in the apparatus III for scrubbing and production of the Fertiliser 2.

The aforesaid flows then traverse for example in back current an apparatus II wherein the heat exchange takes place, necessary for maintaining the temperature of the digester at the values required and simultaneously the extraction in the biogas of the ammonia, eliminating in this way from the liquid the main factor of inhibition of the fermentation. The overall circulation of the liquid is moreover such as to allow a circulation in general daily of the content of the digester from the base to the surface and a homogenisation of the liquid through the conduits and the process apparatuses. The apparatus for heat exchange and for extraction in gas phase of the ammonia is typically a gas/liquid column or contactor provided with exchanger or lining heated by heating fluid 8 optionally coming from the cogeneration plant F and preferably a thin-film scraped-wall column given the viscous nature of the circulating fluid and the low load losses for the circulation of the biogas. Part of the recirculated liquid is sent to the storage of the Fertiliser 1, part returns to the fermenter after having in part contributed in IV to the dispersion of the organic substrates 1 fed.

The biogas exiting from the column II and containing ammonia is sent to one or more columns III where it is put in contact with a recirculating solution of ammonia phosphate or sulphate constituting the Fertiliser 2, wherein sulphuric or phosphoric acid 11 is fed and optionally water. The Fertiliser 2 is drawn from this recirculation for the storage 12. By adjusting the concentration of the acid and of the possible scrubbing water it is possible to adjust the concentration of the Fertiliser 2.

The biogas exiting from said column III without ammonia is in part recycled to the digester and in part 4 sent to the motors of the cogeneration plant F where electrical energy is produced for the plant and the heat in the form of hot water 8 for the thermostatting of the process.

The tanks for storage of the fertilisers produced are of a size functional to the quantity and their period of use: Fertiliser 1 for the period prior to sowing and Fertiliser 2 for the period post sowing.

### Example 1

The example relates to the process described and its use on two lines in a centre serving agricultural land. The centre deals overall with the following organic substrates, coming from the cycle of production and consumption of foods:
- 12,000 t/year of fowl droppings
- 20,000 t/year of filter-pressed biological sludge of an agricultural and food firm
- 50,000 t/year of filter-pressed municipal sludge in order to obtain:
- 100,000 t/year of mixed organic Fertiliser 1 in aqueous dispersion
- 5,850 t/year of Fertiliser 2, consisting of a 20% ammonia sulphate solution.

The reception of the organic substrates is made up of two square vessels, 500 m3 each, having side of 10 metres, served by a robotised gantry crane having width of 20 metres and running length of 40. One side of each vat is situated at two discharge doors in a plane superelevated by 5 metres with respect to the base of the vats. Said doors are equipped with a safety system to avoid the possible falling into the vats of the staff present during their opening. The gantry crane is equipped with clamshell buckets with complete closure and is programmed to mix one with the other the organic substrates received, maintain the spaces under the discharge doors clear and feed the disperser with the organic substrates. In the example described the two lines are identical, with the exclusion of the disperser, in that each line uses a different type of disperser.

The organic substrates described above are roughly mixed one with the other by the automatic gantry crane before sending, in a quantity equal to 50%, to each line. The sending of the organic substrates to each line takes place for 8,000 hours/year with a flow rate of 6 t/hour per line.

In one line the disperser is similar to a pulper for the creation of a suspension of fibres of clean cellulose from recycled paper, wherein the organic substrates described above and the Fertiliser 1 are fed directly by the gantry crane. In the second line the disperser is made with an apparatus similar to a ball mill, fed in a similar way. At the outlet of the dispersers the Fertiliser 1, with the micronised organic substrates dispersed, is fed to the digesters together with the recirculated product. The hourly flow rate of the Fertiliser 1 is 6 m3/hour, the volume of the single digester is 5000 m3 and the working temperature equal to 55°C. The temperature is maintained by heating the recycled product of the digester to slightly over 56°C by means of two scraped thin-film columns, having a diameter of approximately 2 m, heated in the lining using the heating fluid coming from the cogeneration unit wherein the biogas produced is used.

The biogas which passes in back current inside said columns extracts the ammonia from the recirculated product of the fertiliser 1 to then release it subsequently in two columns with filling in series. In these columns the fertiliser 2 is recirculated in the presence of acid, in one column, and of water, in the other, in order to produce a purified biogas to be recirculated to the digester and to be fed to the cogeneration plant.

The cogeneration plant has an electrical power of 500 kWe and an availability of heat with the fumes and the cooling water of 800 kWt per line.

The Fertiliser 1 coming from each line is fed to the storage tank, having capacity of 50,000 m3 with floating roof, and similarly the Fertiliser 2 in a 6000 m3 tank.

The floating roof of the tanks indicated above has the possibility of functioning as a gasometer with a capacity for receiving biogas equal to a volume of 500 m3 for each tank.

The two tanks of Fertiliser 1 and the tank of Fertiliser 2 are connected to the station of loading of the vehicles for the transport of the Fertilisers. For the Fertiliser 1 service tanks are provided for the addition of possible additives before loading.

The following are the methods of dimensioning of the centre serving an area of agricultural land which comprises five municipalities in a territorial region represented by the provinces of Milan and Pavia where organic substrates coming from said territorial region are collected.

The arable surface area served by the centre is 10,120 hectares with the following crops: Rice: 7,800 ha, Corn: 1,220 ha, Wheat: 563 ha, Soy: 211 ha, Other: 326 ha.

The following livestock farms are present on the territory: No. of heads of poultry: 39,277, No. of heads of cattle: 2,674, No. of heads of pigs: 5,188.

The net requirement of nutrient elements required by the Centre can be estimated as the difference between the quantity of the various elements required by the crops present in the territory and that available in the territory and in the particular case: N 1471 - 422 = 1049 t/a, P205 601-84 = 517 t/a, K20 703-113 = 590 t/a.

As can be noted most of the nutrient elements are dispersed in the food cycle and have to be recovered far from agriculture from production and processing systems and zones where moreover their removal represents a problem.

### Second embodiment

The process of the invention, illustrated schematically in Figure 2, operates as follows:
the organic substrates 1 are fed into a disperser IV which allows their micronisation and mixing with the concentrate exiting from the evaporator II via the line 7.

It is possible in this phase to add in the mixer IV a further part of the digested product (Fertiliser 1) coming from the digester and/or other additives, enzymes and process fluids not indicated in the drawing.

The digester I is fed via the line 3, produces the Fertiliser 1 and is constituted by a tank, suitable for guaranteeing the necessary retaining time, typically 20 days, for the thermophilic digestion of the organic substrates dispersed at a temperature higher than that required by pasteurisation, in general 55°C, and for the production of biogas. Said fermenter I can be equipped with a ricirculation of liquid and of biogas for a better mixing and is insulated.

From it the digested product produced (Fertiliser 1) is taken via the line 5 and a flow of digested product via the line 2.

The digested product 2 can be preheated by means of an apparatus V for heat exchange and sent to an evaporator II for the stripping in gas phase of the ammonia.

Said apparatus II is a typically a gas/liquid column or contactor equipped with exchanger or lining heated with hot fluid or vapour 8 coming from the cogeneration plant F whereto it returns a colder fluid or condensate 9.

II is preferably a thin-film scraped-wall column given the viscous nature of the circulating fluid and the need for low load losses for the circulation of the stripping gas/vapour.

The flow 6 exiting from the evaporator is made up of an insert gas, for example carbon dioxide, present in the digested product or of biogas and vapour containing the stripped ammonia.

The flow 6, 6bis enters the apparatus III for scrubbing and neutralisation of the ammonia with acid and is recycled via the line 13, 13bis to the evaporator II.

The apparatus III for scrubbing of the ammonia can be a column or preferably, as indicated in Figure 2, a Venturi scrubber whereon an aqueous stream 10 is made to circulate wherein sulphuric acid 11 and water 16 is fed via the flow 15.

The recycle 6, 6bis 13, 13bis is carried out with the vapour produced by the evaporator and with the gases released by the digested product or alternatively with the same biogas produced by the digester 4 fed into the scrubber via the flow 4bis and with a recycle ratio such as to maintain the evaporator/scrubber system at high temperatures and between 65 and 95°C and at atmospheric pressure or slightly pressurised.

The scrubber III therefore produces alternatively a discharge 14 which can be combined with the biogas 4 creating the flow 17 or alternatively all the scrubbed biogas 14, 17.

From the scrubber III exits a concentrated flow 12 of ammonia sulphate which constitutes the Fertiliser 2.

The combined use of the evaporator II and of the scrubber III in closed cycle allows the use in varied form of all the residual heat produced by internal combustion engines, to extract at high temperature the ammonia as Fertiliser 2 and to maintain the temperature of the digester around 55°C, useful for the digestion and for the pasteurisation of the Fertiliser 1.

### Example 2

The example relates to the process described and its use on two lines in a centre serving agricultural land.

Each line is fed with 4600 kg/h (110 t/g) of organic substrates at ambient temperature, homogenised and containing 650 kg/h of dry substance, approximately 50 kg/h of ammonia and organic nitrogen.

Said substrates are mixed in IV with approximately 13000 kg/h of hot fluid 7 at 78°C, coming from the evaporator II, in order to be fed to the digester, having a working volume of 4500 m3, with the flow 3 at approximately 64°C in such a way as to compensate the dispersions of the insulated digester and maintain the temperature at around 55°C.

The digester produces 350 g/h of biogas and 4350 kg/h of digested product constituting the Fertiliser 1 and a flow rate of 13000 kg/h is taken therefrom which is preheated in V to 60°C and fed to the evaporator II.

Between the evaporator II and the scrubber III, by acting on the discharge 14, a flow of recirculation is produced of approximately 25000 m3/h of gas constituted by carbon dioxide and vapour which transfers around 40 kg/h of ammonia to the scrubber where it is neutralised with sulphuric acid to give 500 kg/h of a 30% aqueous solution of ammonia sulphate.

The evaporator uses approximately 500 kg/h of vapour produced with the fumes at 470°C exiting from a 550 kWe engine fed by the biogas produced by the line and operates at atmospheric pressure.

The temperature of the evaporator-scrubber system is 78 - 80°C.

### Example 3

The operation is as in the previous case but feeding the biogas in the recycling between evaporator and scrubber activating the flow 4bis and, with a discharge equal to 5% of the recycling, a recycled flow rate of inert gas of around 12000 m3/h mainly constituted by biogas and vapour.

From the recycling the biogas is extracted which is sent to internal combustion engines.

In this case the recovery of the ammonia is approximately 34 kg/h, with production of 460 kg/h of a 28% solution of ammonia sulphate, with temperatures in the evaporator and in the scrubber around 75 and 76.5°C and pressure around atmospheric pressure.

The increase in the temperature in the evaporator together with the possibility of management of the recycling has therefore a positive effect on the extraction of the ammonia making the plant flexible to the various types of waste, without entailing increases of energy used, supplying the heat for the performance of the digestion and lowering the concentration of ammonia in the digester.

The availability of centres serving areas of agricultural land distributed over the territory allows a virtuous cycle to be closed which had been interrupted with the industrialisation no longer on the scale of the single farm but on a regional scale with enormous advantages both economic and environmental.

The invention comprises obviously also possible variations on the diagrams of flow of the embodiments described above, within reach of the persons skilled in the art. For example single apparatuses could be replaced by combinations of several apparatuses or a condenser and a vacuum pump could be added in order to operate in a light vacuum without thereby departing from the spirit and from the objects of the invention.

The process described can be advantageously implemented in centres for the recovery of the nutrient elements removed by harvesting from agricultural land, wherein the waste coming from the cycle of production and consumption of foods (organic substrates) is collected during the year. Thus two fertilisers are obtained which are stored at the end of their use in the period of fertilisation: a first fertiliser containing also stable organic substance can be used preferably before sowing while a second fertiliser of inorganic type can be used also after sowing.

Naturally the invention is not limited to the particular embodiments described and illustrated above, but numerous detailed modifications can be made thereto without departing from the scope of the invention, as defined by the annexed claims.

## Claims

1. A process for the recycling of nutrient elements and for the preparation of fertilisers from organic substrates from waste coming from the cycle of production and consumption of foods using a digester, an evaporator for the extraction of the ammonia, an absorber of the ammonia and a mixer, **characterised by**:
1.1 - anaerobic fermentation as a continuous process in the digester at a temperature of around 55°C for the production of a digested product (Fertiliser 1) and of biogas;
1.2 - placing in contact in the evaporator a flow of digested product exiting from the digester with a recycling of gas for the extraction of the ammonia, supplying the heat necessary for the evaporation;
1.3 - recycling of the hot digested product exiting from the evaporator to the digester after mixing in the mixer with the organic substrates fed with the aim of improving the homogenisation in the digester and maintaining the temperature thereof;
1.4 - absorption of the ammonia with a fluid containing sulphuric/phosphoric acid for the production of ammonia sulphate/phosphate for the production of a Fertiliser 2;
1.5 - use of the biogas in internal combustion engines for the production of electrical energy and of the heat necessary for the process.

2. Process according to claim 1, wherein the evaporator operates at temperatures slightly higher than that of the digester and preferably between 55 and 60°C and wherein a flow of biogas exiting from the digester is used for the extraction of the ammonia and which, after having traversed the evaporator and the absorber, is recycled to the digester.

3. Process according to claim 1, wherein the evaporator operates at temperatures higher than 60°C and wherein a flow of gas is used for the extraction of the ammonia made up of biogas, carbon dioxide or inert types recycling in closed cycle between evaporator and absorber of the ammonia operating at a temperature close to that of the evaporator.

4. Process according to any one of the preceding claims, wherein the digester is an insulated tank provided with a recirculation of liquid able to ensure a daily liquid exchange and a recirculation of biogas in such a quantity as to ensure the extraction of the ammonia from the recirculating liquid.

5. Process according to any one of the preceding claims, wherein the evaporator is an apparatus preferably external to the digester to extract in gas phase the ammonia and simultaneously supplying heat, said apparatus being a column and preferably a scraped thin-film column equipped with heated lining.

6. Process according to any one of the preceding claims, wherein the absorber of the ammonia extracted from the evaporator is a column or a Venturi scrubber in which a concentrated solution of an ammonium salt recirculates, which constitutes the Fertiliser 2, whereto acid of adequate concentration is added in a continuous process, sufficient for neutralising the ammonia extracted, the temperature of this fluid being such as to avoid or control the transfer of water from the first to the second circulating fluid and vice versa.

7. Process according to any one of the preceding claims, wherein the mixer for homogenising the organic substrates with the recycling of the digester is a ball mill or a pulper.

8. Process according to any one of the preceding claims, wherein the water for cooling of the hot fumes of the cogeneration plant is used to supply heat to the evaporator for the extraction of the ammonia.

9. Process according to the preceding claims equipped with a section for collection of said organic substrates in the solid, liquid or sludgy state in one or more vessels in a closed environment in a vacuum connected to a system of deodorisation of the air.

10. Process according to the preceding claims equipped with storage of the Fertiliser 1 in a tank of sufficient capacity for supplying the Fertiliser 1 in the period of fertilisation before sowing, and storage of the Fertiliser 2 in a tank of sufficient capacity for supplying the Fertiliser 2 in the period of fertilisation also subsequent to sowing.

11. Use of the process according to any one of the preceding claims in centres for the recovery of the nutrient elements removed by harvesting from agricultural land, wherein the waste coming from the cycle of production and consumption of foods is collected during the year for the production of a first fertiliser containing stable organic substance, usable before sowing, and a second inorganic ammonium fertiliser usable also after sowing.
